(19) Europäisches Patentamt
European Patent Office
Office européen des brevets

(11) Veröffentlichungsnummer: **0 023 981**
**B1**

(12) # EUROPÄISCHE PATENTSCHRIFT

(45) Veröffentlichungstag der Patentschrift: **08.12.82**

(51) Int. Cl.³: **C 07 C 125/073**, C 07 C 125/077

(21) Anmeldenummer: **80104091.6**

(22) Anmeldetag: **15.07.80**

(54) **Verfahren zur Herstellung von Methylen-bis-phenylcarbaminsäureestern und Polymethylen-polyphenylcarbamin-säureestern.**

(30) Priorität: **03.08.79 DE 2931554**

(43) Veröffentlichungstag der Anmeldung: **18.02.81 Patentblatt 81/7**

(45) Bekanntmachung des Hinweises auf die Patenterteilung: **08.12.82 Patentblatt 82/49**

(84) Benannte Vertragsstaaten: **AT BE DE FR GB IT NL**

(56) Entgegenhaltungen:
**EP-A-0 014 379**
**US-A-2 946 768**
**US-A-3 714 280**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Nestler, Gerhard, Dr., Rubensstrasse 25, D-6700 Ludwigshafen (DE)**

**EP 0 023 981 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Methylen-bis-phenylcarbaminsäureestern und Polymethylen-polyphenylcarbaminsäureestern

Die Erfindung betrifft ein neues Verfahren zur Herstellung von Methylen-bis-phenylcarbaminsäureestern und von Polymethylen-polyphenylcarbaminsäureestern durch Umsetzung von N-Phenylcarbaminsäureestern mit Acylalen in Gegenwart von Säuren.

Methylen-bis-phenylcarbaminsäureester und davon abgeleitete höhere Homologe sind wertvolle Ausgangsprodukte für die Herstellung von Methylen-bis-phenylisocyanaten und den entsprechenden Polymethylenpolyphenylisocyanaten, die bekanntlich für die Herstellung von Polyurethanen Verwendung finden (DE-OS 26 35 490). Die im Handel erhältlichen Isocyanate dieser Art werden allgemein durch Phosgenierung der bei der Kondensation von Anilin mit Formaldehyd in Gegenwart von wäßrigen Säuren erhältlichen Amine hergestellt.

Methylen-bis-phenylcarbaminsäureester werden durch Umsetzung der Anilin-Formaldehyd-Kondensationsprodukte mit Chlorameisensäureestern in Gegenwart von Basen oder durch Umsetzung der entsprechenden Isocyanate mit Alkoholen erhalten.

Diese Verfahren haben den Nachteil, daß die Umsetzung mit dem stark toxischen Phosgen aus Sicherheitsgründen eine aufwendige Technik erfordert. Außerdem wird durch die Abtrennung der bei den einzelnen Umsetzungen einerseits benötigten und andererseits als Nebenprodukt anfallenden Säuren die Umwelt stark belastet.

Methylen-bis-phenylcarbaminsäureester lassen sich auch durch Umsetzung von Methylen-bis-nitrophenyl mit Alkoholen und Kohlenmonoxid herstellen (s. DE-AS 15 68 044). Da die Darstellung der hierfür benötigten Nitroverbindung große Schwierigkeiten bereitet, hat dieses Verfahren keine technische Bedeutung erlangt.

In der US-PS 29 46 768 wird der Vorschlag gemacht, die gewünschten Carbaminsäureester durch Kondensation von Phenylcarbaminsäureestern mit Formaldehyd oder Formaldehyd abgebenden Verbindungen in Gegenwart von wäßrigen Säuren herzustellen. Bei diesen bekannten Verfahren neigt der Formaldehyd zur Reaktion am Stickstoffatom des Carbaminsäureesters, so daß 15 bis 50 Gew.-% an unerwünschten N-C-verknüpften Produkten entstehen (s. DE-AS 28 37 379, Seite 3, Zeilen 5 bis 13). Ein analog Beispiel 2 der US-PS 29 46 768 durchgeführtes Vergleichsbeispiel (s. unten Beispiel 1) bestätigt diesen Sachverhalt. Außerdem werden noch andere Nebenprodukte gebildet, beispielsweise Amine, die durch saure Hydrolyse des Carbaminsäureesters entstehen.

Da es kein Verfahren gibt, mit dem man die über das Stickstoffatom verknüpften Produkte, die bei der Pyrolyse keine Isocyanate liefern, abtrennen könnte, sind die nach dem Verfahren der US-PS 29 46 768 hergestellten Reaktionsgemische zur Herstellung von Isocyanaten ungeeignet. Dieses Verfahren ist daher in technischer und ökonomischer Hinsicht unbefriedigend.

In der DE-OS 28 32 379 wird ein verfahren zur Umlagerung dieser unerwünschten Nebenprodukte zu Methylen-bis-phenylcarbaminsäureestern und davon abgeleiteten höheren homologen Polymethylen-polyphenylcarbaminsäureestern beschrieben. Es besteht darin, daß die nach dem Verfahren der US-PS 29 46 768 hergestellten Reaktionsgemische unter praktisch wasserfreien Bedingungen bei 50 bis 170° C mit starken Protonensäuren oder Lewissäuren umgesetzt werden.

Dieses technisch schwierige und zweistufige Verfahren ist nachteilig, weil zunächst nach der US-PS 29 46 768 aus Phenylcarbaminsäureester und Formalin in Gegenwart von großen Mengen wäßriger Säuren ein Kondensationsprodukt hergestellt wird, welches von der Säure befreit und getrocknet werden muß und schließlich unter wasserfreien Bedingungen wieder mit großen Mengen an Säure, die nach Ablauf der Reaktion vollständig zu entfernen sind, umgesetzt wird. Die Säuremengen und die gebildeten Nebenprodukte, wie die bei der sauren Hydrolyse entstehenden Amine, stellen eine gravierende Abwasserbelastung dar.

In der US-PS 37 14 280 wird ein Verfahren zur Herstellung von Methylen-bis-phenylverbindungen beschrieben, bei dem man Phenylverbindungen mit Estern aliphatischer Säuren in Gegenwart von Bortrifluorid umsetzt. N-Phenylcarbaminsäureester werden als Ausgangsstoffe nicht erwähnt. Bei der Umsetzung von Toluol mit Methylendiacetat und Bortrifluorid (s. Beispiel IV) wird in schlechter Ausbeute ein Gemisch aus 6 Isomeren des Ditolylmethans erhalten.

Es wurde nun gefunden, daß man die genannten Schwierigkeiten bei der Herstellung von Methylen-bis-phenylcarbaminsäureestern und Polymethylenpolyphenylcarbaminsäureestern vermeiden kann, wenn man N-Phenylcarbaminsäureester der Formel

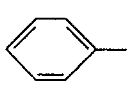—NH—COOR'

in der R' einen Alkylrest mit 1 bis 3 C-Atomen bedeutet und der Phenylrest in den o- und/oder m-Stellungen durch Methyl- oder Methoxygruppen oder durch Halogenatome wie Chlor- oder Bromatome substituiert sein kann, in Gegenwart einer Säure mit Acylalen der Formel

$CH_2(OCOR)_2$

in der R einen Alkylrest mit 1 bis 3 C-Atomen bedeutet, bei Temperaturen von 50 bis 150° C umsetzt.

Nach diesem neuen Verfahren werden die Methylen-bis-phenylcarbaminsäureester und Polymethylenpolyphenylcarbaminsäureester aus den N-Phenylcarbaminsäureestern überraschend glatt in guter Ausbeute und mit hoher Reinheit in einer einzigen Stufe erhalten.

Das Reaktionsschema kann für den Fall der Bildung des Methylen-bis-(4-phenylcarbaminsäureesters) aus dem N-Phenylcarbaminsäuremethylester und Diacetoxymethan durch folgende Formeln wiedergegeben werden:

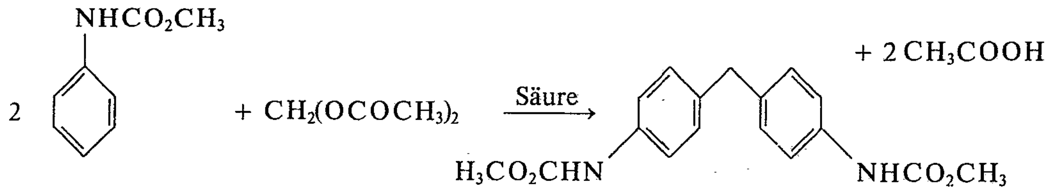


Bei den erfindungsgemäßen Verfahren werden gleichzeitig auch höhere homologe Polymethylenpolyphenylcarbaminsäureester, das sind 3- und mehrere durch Methylenbrücken miteinander verbundene Benzolringe enthaltende Carbaminsäureester, gebildet, da die Acylale in untergeordnetem Maße auch mit bereits gebildetem Methylen-bis-phenylcarbaminsäureester reagieren.

Geeignete N-Phenylcarbaminsäureester sind beispielsweise N-Phenylcarbaminsäuremethyl-, äthyl- oder propylester, N-o-Tolylcarbaminsäuremethyl- oder äthylester, N-2,6-Dimethylphenylcarbaminsäuremethylester oder N-o-Chlor-phenylcarbaminsäureäthylester.

Die Acylale bilden unter den Umsetzungsbedingungen praktisch keinen freien Formaldehyd und schließen bei der Umsetzung nach der Erfindung die Bildung von Reaktionswasser aus. Vorzugsweise verwendet man solche Acylale, in denen der Alkylrest mit dem Alkanol übereinstimmt, der dem Carbaminsäureester zugrunde liegt.

Beispielsweise sei als Acylal das Diacetoxymethan genannt.

Man führt die Umsetzung der Ausgangsstoffe in Gegenwart einer Säure be Temperaturen von 50 bis 150° C, vorzugsweise 90 bis 140° C, insbesondere 90 bis 120° C durch.

Das Molverhältnis Acylal : Carbaminsäureester liegt allgemein bei 1 : 0,5 bis 1 : 10, vorzugsweise bei 1 : 1,5 bis 1 : 3. Will man hauptsächlich Methylen-bisphenylcarbaminsäureester unter weitgehender Vermeidung der Bildung von Polymethylenpolyphenylcarbaminsäureestern herstellen, so verwendet man vorzugsweise ein Verhältnis von 1 : 4 bis 1 : 8.

Als Säuren, die man z. B. in Mengen von 1 bis 100, vorzugsweise 10 bis 60 Mol-%, bezogen auf den Carbaminsäureester, anwendet, sind z. B. Phosphorsäure, Schwefelsäure, Alkylsulfonsäure wie Methansulfonsäure oder Arylsulfonsäure wie p-Toluolsulfonsäure geeignet. Entsprechend einer besonders vorteilhaften Ausführungsform der Erfindung verwendet man eine starke Säure, die vom Reaktionsgemisch destillativ abgetrennt werden kann, beispielsweise Trifluormethansulfonsäure. Auf diese Weise entfällt die Aufarbeitung des Reaktionsgemisches mit Wasser oder Basen und die Säure kann direkt erneut der Reaktion zugeführt werden.

Nach einer weiteren vorteilhaften Ausführungsform der Erfindung verwendet man als Säuren stark saure organische Kationenaustauscher, beispielsweise sulfonsaure Austauscherharze. Diese Ionenaustauscher werden nach an sich bekannten Methoden entweder im Reaktionsgemisch suspendiert oder in einem Festbett angeordnet.

Man führt das erfindungsgemäße Verfahren vorzugsweise unter möglichst weitgehender Abwesenheit von Wasser, insbesondere in Abwesenheit von Wasser, d. h. unter Verwendung von Säuren durch, die praktisch kein Wasser enthalten. Man kann sowohl ohne als auch in Gegenwart eines nicht wäßrigen Lösungsmittels, beispielsweise Benzol, Methylcyclohexan, Essigsäure, Methanol, Methylacetat, Nitrobenzol, Chlorbenzol, Dichlorbenzol oder chlorierten aliphatischen Kohlenwasserstoffen arbeiten.

Die Umsetzung, die etwa nach 0,5 bis 20 Stunden beendet ist, wird im allgemeinen so durchgeführt, daß man entweder zu einem Gemisch aus dem Carbaminsäureester und der Säure unter Rühren bei der Reaktionstemperatur das Acylal langsam zugibt oder daß man ein Gemisch aus dem Carbaminsäureester, dem Acylal und der Säure unter Rühren erhitzt und die Gemische die entsprechende Zeit auf der Reaktionstemperatur hält. Die Isolierung des Reaktionsproduktes erfolgt nach herkömmlichen Methoden, z. B. durch Extraktion der Säure mit Wasser oder Neutralisation mit einer Base. Das gegebenenfalls vorhandene Lösungsmittel und unumgesetzte Ausgangsprodukt werden mittels einer Vakuumdestillation abgetrennt.

Die Kondensation der Phenylcarbaminsäureester mit den Acylalen kann in Einzelansätzen oder als kontinuierliches Verfahren durchgeführt werden.

Ein wesentlicher Unterschied, durch den sich das erfindungsgemäße Verfahren von den Verfahren der US-PS 29 46 768 und DE-OS 28 32 379, bei denen Formaldehyd oder Formaldehyd bildende Verbindungen verwendet werden, so daß stets Wasser im Reaktionsgemisch vorliegt, unterscheidet,

besteht darin, daß bei dem erfindungsgemäßen Verfahren kein Formaldehyd und somit kein Reaktionswasser gebildet wird. Eine Hydrolyse der Carbaminsäureester zu Aminen, die Bildung von Harnstoffen und die damit verbundene Belastung des Abwassers werden somit unterbunden. Überraschenderweise tritt auch bei Verwendung der Acylale keine Acylierung am N-Atom des Carbinsäureesters auf.

### Beispiel 1 (Vergleichsbeispiel)

Analog Beispiel 2 der DE-PS 10 42 891 wird ein Gemisch aus 183 Teilen Phenylcarbaminsäuremethylester, 500 ml Wasser und 86 Teilen Formalin (30%ig) unter Rühren auf 100°C erwärmt. Dann fügt man 100 ml konzentrierte Salzsäure hinzu. Die Reaktionsgemisch wird anschließend 20 Stunden bei 100°C gerührt. Nach Beendigung der Umsetzung trennt man die wäßrige Phase ab. Das Reaktionsprodukt wird dreimal mit heißem Wasser gewaschen, anschließend wird im Vakuum unumgesetztes Ausgangsprodukt abdestilliert. Der Rückstand wird mittels Hochdruck-Flüssigkeits-Chromatographie (HPLC) analysiert. Er enthält 50% Methylen-bis-phenylcarbaminsäuremethylester, 9% Dreikernprodukt, 16% N-C-verknüpftes Zweikernprodukt und 10% N-C-verknüpftes Dreikernprodukt. Der Rest besteht aus nicht näher identifizierten höherkernigen Verbindungen.

### Beispiel 2 (Vergleichsbeispiel)

Ein Gemisch aus 90 Teilen Phenylcarbaminsäuremethylester, 250 ml Chlorbenzol und 40 Teilen Formalin (30%ig) wird unter Rühren auf 100°C erwärmt, dann fügt man 50 ml konzentrierte Salzsäure hinzu. Das Reaktionsgemisch wird anschließend 20 Stunden bei 100°C gerührt. Nach Beendigung der Umsetzung wird die wäßrige Phase abgetrennt und mit Wasser zweimal gewaschen. Anschließend werden Chlorbenzol und unumgesetztes Ausgangsprodukt abdestilliert. Entsprechend der HPLC-Analyse enthält der Rückstand 49% Methylen-bis-phenylcarbaminsäuremethylester, 12% Dreikernprodukt, 15% N-C-verknüpftes Zweikern- und 9% Dreikernprodukt. Der Rest besteht aus nicht näher identifizierten höherkernigen Verbindungen.

### Beispiel 3

In einem Rührreaktor wird ein Gemisch aus 151 Teilen Phenylcarbaminsäuremethylester, 66 Teilen Diacetoxymethan, 100 Teilen Nitrobenzol und 20 Teilen Trifluormethansulfonsäure unter Rühren auf 100°C erhitzt und 5 Stunden bei dieser Temperatur gerührt. Nach Beendigung der Umsetzung werden im Vakuum das Lösungsmittel und nicht umgesetztes Ausgangsmaterial abdestilliert. Man erhält 149 Teile eines Destillationsrückstandes, der entsprechend der HPLC-Analyse aus 71 Teilen Methylen-bis-phenylcarbaminsäuremethylester, 20% 3-Kernprodukt und 9% höherkernigen Produkten besteht.

### Beispiel 4

In einem Rührreaktor wird ein Gemisch aus 302 Teilen Phenylcarbaminsäuremethylester, 44 Teilen Diacetoxymethan und 50 Teilen ®LEWASORB AC-10 unter Rühren auf 110°C erhitzt und 5 Stunden bei dieser Temperatur gerührt. Nach Beendigung der Reaktion wird der Katalysator abgetrennt und im Vakuum nicht umgesetztes Ausgangsmaterial und die gebildete Essigsäure abdestilliert. Der Destillationsrückstand wird in Toluol umkristallisiert. Man erhält 90 Teile Methylen-bis-(4-phenylcarbaminsäuremethylester).

®LEWASORB AC-10 ist ein im Handel erhältlicher gelartiger sulfonsaurer Kationenaustauscher aus Styrol-Divinylbenzol (8%)-Copolymerisat mit einer Korngröße von 0,01 bis 0,2 mm und einer Totalkapazität von etwa 4,2 mval/g Trockensubstanz.

### Beispiel 5

In einem Rührreaktor wird ein Gemisch aus 151 Teilen Phenylcarbaminsäuremethylester, 66 Teilen Diacetoxymethan, 120 Teilen Nitrobenzol und 50 Teilen ®LEWATIT SPC-108 unter Rühren auf 100°C erhitzt und 5 Stunden bei dieser Temperatur gerührt. Nach Beendigung der Umsetzung wird der Katalysator abgetrennt, und dann werden im Vakuum Nitrobenzol und nicht umgesetztes Ausgangsmaterial abdestilliert. Man erhält 142 Teile eines Destillationsrückstandes, der entsprechend der HPCL-Analyse aus 76% Methylen-bis-phenylcarbaminsäuremethylester, 16% 3-Kernprodukt und 8% höherkernigen Produkten besteht.

®LEWATIT SPC-108 ist ein im Handel erhältlicher makroporöser sulfonsaurer Kationenaustauscher aus Styrol-Divinylbenzol (8%)-Copolymerisat, Korngröße 0,3—1 mm, Totalkapazität ca. 4,2 mval/g Trockensubstanz.

## Patentansprüche

1. Verfahren zur Herstellung von Methylen-bis-phenylcarbaminsäureestern und Polymethylenpoly-phenylcarbaminsäureestern, dadurch gekennzeichnet, daß man N-Phenylcarbaminsäureester der Formel

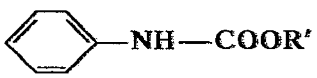

in der R' einen Alkylrest mit 1 bis 3 C-Atomen bedeutet und der Phenylrest in den o- und/oder m-Stellungen durch Methyl- oder Methoxygruppen oder durch Halogenatome wie Chlor- oder Bromatome substituiert sein kann, in Gegenwart einer Säure mit Acylalen der Formel

$CH_2(OCOR)_2$

in der R einen Alkylrest mit 1 bis 3 C-Atomen bedeutet, bei Temperaturen von 50 bis 150°C umsetzt.

2. Verfahren nach Anspruch 1, dadurch gekennzeichnet, daß man die Umsetzung in Abwesenheit von Wasser durchführt.

3. Verfahren nach Anspruch 1, dadurch gekennzeichnet, daß man als Säure Trifluormethansulfonsäure verwendet.

4. Verfahren nach Anspruch 1, dadurch gekennzeichnet, daß man als Säuren stark saure organische Kationenaustauscher verwendet.

## Claims

1. A process for preparing methylene-bis-phenyl-carbamic acid esters and polymethylene-poly-phenylcarbamic acid esters, wherein an N-phenylcarbamic acid ester of the formula

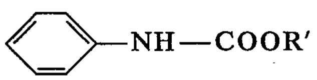

where R' is alkyl of 1 to 3 carbon atoms and the phenyl radical may be substituted in the o- and/or m-positions by methyl or methoxy groups or by halogen atoms, such as chlorine or bromine atoms, is reacted with an acylal of the formula

$CH_2(OCOR)_2$

where R is of 1 to 3 carbon atoms, in the presence of an acid at from 50° to 150° C.

2. A process as claimed in claim 1, wherein the reaction is carried out in the absence of water.

3. A process as claimed in claim 1, wherein trifluoromethanesulfonic acid is used as the acid.

4. A process as claimed in claim 1, wherein a strongly acidic organic cation exchanger is used as the acid.

## Revendications

1. Procédé pour la préparation d'esters d'acide méthylène-bis-phénylcarbamique et d'esters d'acide polyméthyléne-polyphénylcarbamique, caractérisé en ce qu'on fait réagir, à une température comprise entre 50 et 150° C, un ester d'acide N-phénylcarbamique de formule

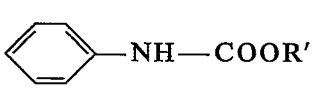

dans laquelle R' représente un radical alcoyle à 1—3 atomes de C et le radical phényle peut être substitué, dans les positions o et/ou m, par des groupes méthyle ou méthoxy ou par des atomes

d'halogène, tels que des atomes de chlore ou de brome, en présence d'un acide, avec des acylals de formule

$CH_2(OCOR)_2$

dans laquelle R représente un radical alcoyle à 1 – 3 atomes de C.

2. Procédé selon la revendication 1, caractérisé en ce qu'on mène la réaction en l'absence d'eau.

3. Procédé selon la revendication 1, caractérisé en ce qu'on utilise, en tant qu'acide, l'acide trifluorométhane-sulfonique.

4. Procédé selon la revendication 1, caractérisé en ce qu'on utilise, en tant qu'acides, des échangeurs de cations organiques fortement acides.